# FASCICULE DE BREVET EUROPEEN

(11) **EP 0 925 763 B1**
(45) Date de publication et mention de la délivrance du brevet: **31.03.2004**
(21) Numéro de dépôt: 98403049.4
(22) Date de dépôt: 04.12.1998
(51) Int. Cl.: A61F 2/06

(54) **Ensemble pour la mise en place d'un implant dans un conduit interne d'un corps**
Einheit zum Einsetzen eines Implantates in ein Gefäss im Körper
Assembly for placing an implant into an internal body conduit

(30) Priorité: 19.12.1997 FR 9716147
(43) Date de publication de la demande: 30.06.1999
(73) Titulaire: B. Braun Medical SAS, 92100 Boulogne Billancourt (FR)
(72) Inventeur: Forber, Simon John, 86170 Yversay (FR)
(74) Mandataire: Lerner, François

(56) Documents cités:
- EP-A- 0 696 447
- EP-A- 0 737 451

## Description

Le domaine de l'invention est celui des dispositifs utilisés pour la mise en place d'endoprothèses dans un conduit interne d'un corps, et notamment dans un vaisseau.

Les endoprothèses présentent un axe et comprennent une structure propre à présenter un premier état radialement déployé dans une situation implantée dans le conduit, ou un second état radialement restreint en situation de mise en place, par exemple à l'intérieur d'une gaine d'introduction intraluminale. En pratique, de tels implants pourront consister notamment en un dispositif d'occlusion ou vasculaire, un filtre sanguin à pattes, voire un stent pour le traitement d'une sténose ou une prothèse pour le traitement d'un anévrisme.

On rencontre actuellement parfois des difficultés pour disposer l'implant dans son état resserré à l'intérieur de sa gaine introductrice, en particulier lorsqu'il est de petites dimensions. De même, dans le cas de la mise en place d'un implant de type temporaire ou temporaire/définitif à l'intérieur d'un conduit, il est nécessaire de pouvoir tenir ou détacher l'implant de son système introducteur, voire de pouvoir venir le récupérer après l'avoir largué.

Le document EP-A-0 737 451 divulgue un ensemble pour la mise en place d'un implant dans un conduit anatomique, comprenant un implant ayant une structure propre à présenter un premier état radialement déployé ou un second état radialement restreint,
et un dispositif de retenue ou de libération de l'implant, comprenant un élément allongé amovible, cet élément présentant, à l'extrémité distale, une tête élargie. La largeur de la tête élargie est adaptée au diamètre interne d'un creux, formé par l'implant dans l'état restreint, de sorte que la tête élargie retienne l'implant. En situation de mise en place de l'implant dans le conduit, l'implant prend l'état radialement déployé et libère la tête élargie de l'élément allongé.

L'invention propose une solution simple, sûre, adaptable à différents types d'implant, facile à réaliser et peu coûteuse.

Suivant cette solution, le dispositif de retenue ou de libération de l'implant, comprend au moins un premier et un second éléments allongés amovibles ayant une section déterminée et s'étendant essentiellement parallèlement l'un à l'autre, le premier élément présentant, vers une extrémité distale, une excroissance latérale ayant une longueur radiale, tandis que l'autre élément en est dépourvu, la dimension cumulée en section des deux éléments à l'écart de leur extrémité distale et la longueur radiale de ladite excroissance latérale étant inférieures au diamètre interne de la zone tubulaire de l'implant pour que les éléments passent à travers par leur extrémité distale, en situation de mise en place de l'implant dans le conduit, tout en étant adaptés pour en être retirés suite à cette mise en place.

Afin d'améliorer la retenue de l'implant par le dispositif de retenue/libération, et dans le but de faciliter l'utilisation de ce dispositif, les premier et second éléments allongés se présenteront de préférence comme deux longs fils disposés glissants dans un cathéter avec l'excroissance du premier élément alors située à l'extérieur dudit cathéter, le cathéter et les fils ayant une longueur suffisante pour être manoeuvrés depuis l'extérieur du corps du patient, alors que l'implant est dans le cond uit.

De façon à améliorer le glissement axial des éléments allongés à l'intérieur de la gaine sans influence néfaste sur le retenue de l'implant, au moins un des deux fils présentera de préférence, sur la majeure partie de sa longueur, une section strictement inférieure à la moitié du diamètre interne du cathéter, l'autre fil pouvant alors présenter, sur toute sa longueur, une section inférieure ou égale à la moitié du diamètre interne dudit cathéter, de telle sorte que la somme des sections des deux fils soit strictement inférieure au diamètre interne du cathéter.

Dans le même but, les deux fils auront par ailleurs avantageusement une section circulaire et présenter chacun un diamètre strictement inférieur à la moitié du diamètre interne du cathéter sur toute leur longueur à l'exclusion d'une zone proche de leur extrémité distale où leur diamètre sera de préférence supérieur ou égal à la moitié du diamètre interne dudit cathéter.

Toujours dans le but de faciliter la mise en place de l'implant, l'ensemble pourra comprendre en outre une gaine ayant un diamètre interne supérieur au diamètre externe du cathéter et de la zone tubulaire de l'implant, propre à y loger l'implant dans son état radialement restreint, le cathéter et les fils constituant alors un moyen de manoeuvre suffisamment rigide pour assurer un déplacement axial de l'implant pour sa mise en place dans le conduit.

Toujours dans le même but, et pour favoriser le maintien de l'implant en position de tenue de celui-ci, alors que l'extrémité distale des éléments allongés est engagée à travers la zone tubulaire dudit implant, le cathéter pourra être placé sensiblement contre une surface proximale de cette zone tandis que l'excroissance latérale du premier élément pourra être placée contre une surface distale de ladite zone.

Afin de faciliter la retenue de l'implant avant qu'il ne soit détaché de son dispositif de mise en place, le premier élément se présentera de préférence comme une tige sensiblement rectiligne courbée à son extrémité distale en faisant un angle compris entre environ 45° et 90° avec cette tige pour former un court crochet.

Dans le but d'améliorer la mise en place de l'implant à distance, l'ensemble pourra comprendre en outre une poignée présentant une extrémité avant au-delà de laquelle s'étendent les éléments allongés, chaque élément étant fixé à un coulisseau axial, le premier coulisseau auquel est fixé le second élément étant situé devant celui auquel est fixé le premier élément. En particulier, le premier coulisseau sera de préférence muni d'un embout de manoeuvre pour être accessible à la main de l'utilisateur, tandis que le second coulisseau sera inaccessible de l'extérieur de la poignée.

L'invention trouvera son application en particulier dans les cas où l'implant est un filtre sanguin, et de préférence un filtre propre à être implanté soit temporairement, soit temporairement puis définitivement, consécutivement au retrait du dispositif de tenue/libération à l'écart de la zone tubulaire du filtre.

Selon une autre considération, l'implant pourra être un dispositif d'occlusion ou d'athérectomie vasculaire.

L'invention va apparaître encore plus clairement dans la description qui va suivre, faite en référence aux dessins annexés dans lesquels :
- les figures 1 à 4 représentent, vu en coupe, un ensemble conforme à la présente invention et les différentes étapes de mise en place,
- la figure 5 montre en détail la zone tubulaire de l'implant et une partie de son dispositif de mise en place,
- la figure 6 montre un second mode de réalisation pour l'implantation d'un filtre sanguin de type temporaire ou temporaire/définitif, et
- la figure 7 est une variante de réalisation la figure 6,
- la figure 8 est une autre variante de réalisation du dispositif de mise en place de l'implant.

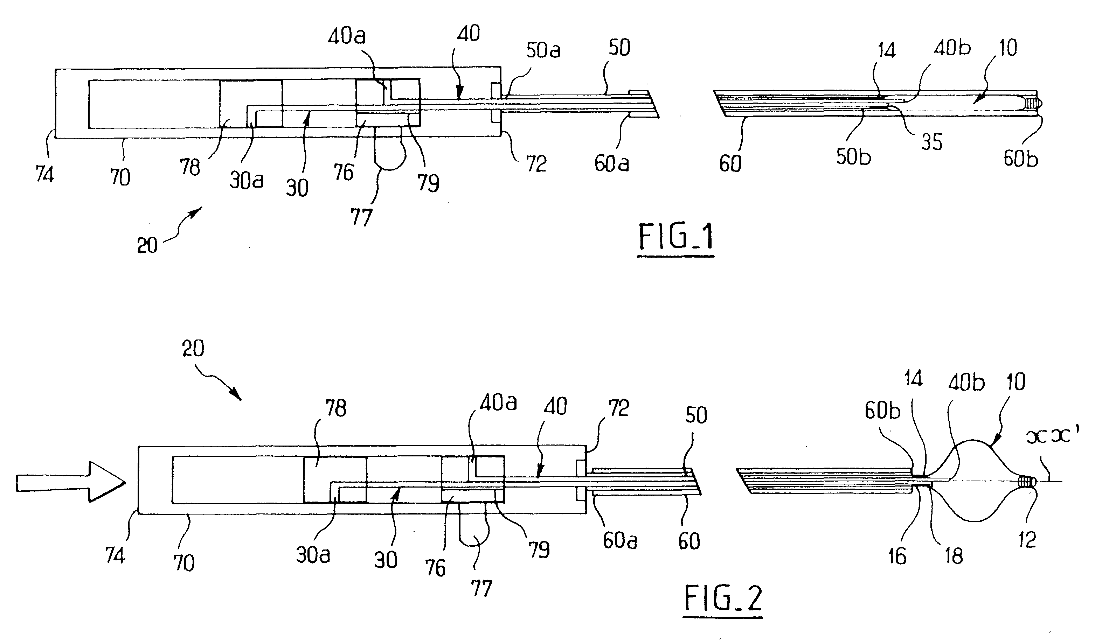

Une endoprothèse 10, qui est ici un dispositif d'occlusion vasculaire (pouvant également faire office de filtre sanguin), est représentée sur les figures 1 à 5. Cette endoprothèse 10 présente un axe longitudinal xx' et comprend au moins une bague (ou anneau) axiale 14 ayant un diamètre intérieur D, une extrémité proximale 16 et une extrémité distale 18.

Cet implant 10 comprend aussi une structure 15 propre à présenter un premier état radialement déployé dans une situation implantée dans le conduit (figures 2 à 4), ou un second état radialement restreint en situation de mise en place (figure 1), par exemple une structure à filaments tressés. Dans son état radialement déployé, il prend la forme d'un panier renflé en partie centrale, resserrée à ses extrémités distale et proximale respectivement par un manchon plein 12 et par la bague creuse 14.

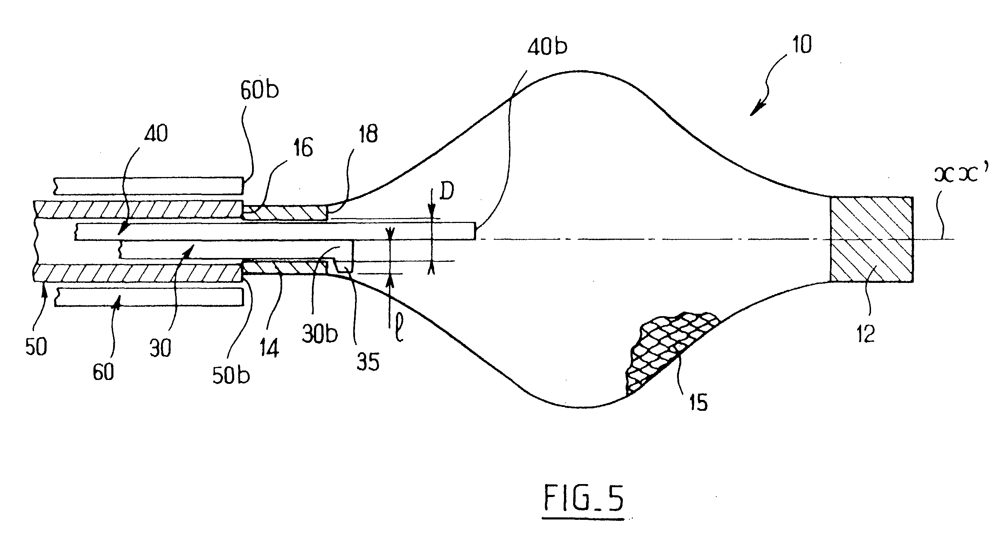

Un dispositif 20 de retenue ou de libération de l'implant 10 est également représenté sur ces figures. Ce dispositif 20 comprend au moins un premier élément 30 et un second élément 40. Ces éléments 30 et 40 sont allongés et mobiles sensiblement selon l'axe xx', et s'étendent sensiblement parallèlement l'un à l'autre. Le premier élément 30 présente, à une extrémité distale 30b, une excroissance latérale 35, typiquement une courbure sensiblement à 90° formant un crochet court par rapport à la longueur axiale (L) de la tige 30 (voir figure 5), tandis que le second élément 40 en est dépourvu, c'est-à-dire en l'espèce exclusivement rectiligne. En particulier, ces éléments allongés 30 et 40 se présentent comme deux fils (ou filaments) de section circulaire (de diamètre de l'ordre de un à quelques dixièmes de millimètre), sensiblement droits, de préférence métalliques, bien que pouvant aussi être en plastique ou en hélice.

La somme des diamètres des deux tiges 30 et 40, prise à l'écart de leur extrémité distale 30b/40b, ainsi que la longueur radiale l du crochet 35, sont inférieures au diamètre intérieur D de la bague 14 de l'implant 10 afin que les éléments 30 et 40 puissent passer à travers elle par leur extrémité distale 30b/40b, tout en étant adaptés pour en être retirés une fois l'implant 10 en place dans le conduit.

Les deux tiges (ou fils) 30/40 sont disposées dans un cathéter 50 à l'intérieur duquel elles peuvent glisser axialement, le crochet 35 du premier fil 30 étant disposé à l'extérieur du cathéter 50, au-delà de son extrémité distale 50b. Ces fils ont une longueur suffisante (typiquement 10 à 20 centimètres) pour pouvoir être manipulés à distance, c'est-à-dire depuis l'extérieur du corps du patient, en vue de permettre la mise en place de l'implant 10 à l'intérieur du conduit considéré.

L'ensemble d'introduction comprend en outre une gaine 60 flexible en matériau plastique biocompatible de diamètre interne supérieur au diamètre externe du cathéter et à celui de l'endoprothèse 10 dans l'état radialement resserré de celle-ci, comme on peut le voir sur la figure 1. La gaine 60 est légèrement plus courte que le cathéter 50, et présente une extrémité distale 60b et une extrémité proximale 60a.

L'ensemble comprend aussi une poignée 70 présentant une extrémité avant 72 à laquelle est fixé le cathéter 50 et au-delà de laquelle s'étendent les fils 30 et 40, ainsi qu'une extrémité arrière 74. A l'intérieur de la poignée 70, le fil 40 est relié à un premier coulisseau 76 et le fil 30 est relié à un second coulisseau 78, le premier coulisseau 76 étant situé en avant du second. Le premier coulisseau 76 est muni d'un embout 77 de manoeuvre sortant de la poignée 70, tandis que le second coulisseau 78 est inaccessible depuis l'extérieur de la poignée 70. Le premier fil 30 peut coulisser à travers un trou 79 du premier coulisseau 76.

A l'aide des figures 1 à 4, nous allons maintenant décrire les étapes de mise en place d'un implant 10.

Supposons que l'implantation s'effectue par voie endoluminale (par exemple par la méthode dite de "SELDINGER").

Sur la figure 1, l'implant 10 est chargé dans la gaine 60 dans son état radialement resserré, vers son extrémité distale 60b. Le cathéter 50 est avancé dans cette gaine 60 de sorte que son extrémité distale 50b prenne appui contre l'extrémité proximale 16 de la bague 14. La poignée 70 est hors du corps du patient et les coulisseaux 76 et 78 sont suffisamment avancés à l'intérieur de la poignée 70 pour que le second fil 40 soit disposé à l'intérieur de l'implant 10, au-delà de la bague 14, et pour que le crochet 35 prenne appui derrière cette bague (à son extrémité distale 18). Dans cette disposition, l'extrémité proximale 60a de la gaine est écartée par rapport à l'extrémité avant 72 de la poignée.

On recule alors la gaine 60 (figure 2) en tenant fixement la poignée 70 (ou on avance la poignée 70 en tenant fixement la gaine 60) de sorte que l'extrémité proximale 60a de la gaine 60 se rapproche de l'extrémité avant 72 de la poignée. Par ce rapprochement, le cathéter 50 pousse axialement l'implant 10 et provoque sa sortie hors de la gaine 60. L'endoprothèse 10 se déploie alors radialement à l'intérieur du vaisseau considéré. Durant cette opération, les coulisseaux 76/78 sont maintenus en place axialement dans la poignée 70, par exemple par un système à cliquet (non représenté).

Si l'endoprothèse 10 doit à ce moment être retirée ou déplacée, il suffira alors de reculer la poignée 70 (sans toucher aux coulisseaux) de façon à la faire rentrer à l'intérieur de la gaine 60, puis de déplacer la gaine.

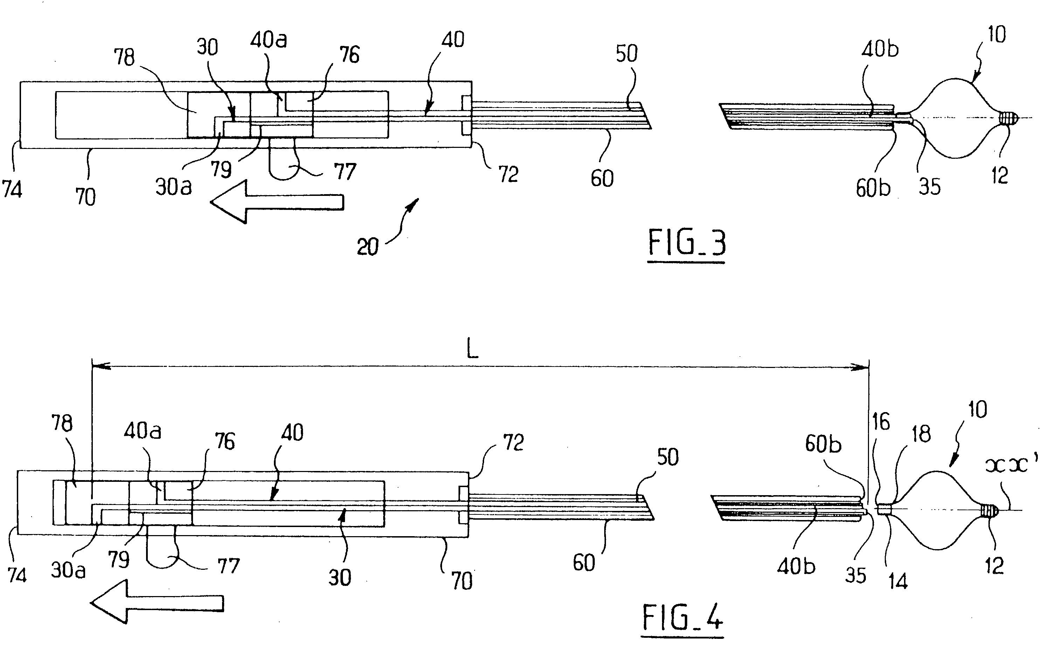

Pour libérer l'implant 10, on recule le premier coulisseau 76 (figure 3) à l'intérieur de la poignée 70 à l'aide de l'embout 77 de manoeuvre, jusqu'à ce qu'il vienne au contact du second coulisseau 78. Le second fil 40 est alors tiré en arrière jusqu'à ce qu'il sorte de la bague 14. Ainsi, seul le premier fil 30 demeure encore dans l'implant 10, lequel reste retenu au dispositif 20 de mise en place par l'intermédiaire du crochet 35.

Pour libérer totalement l'implant 10, on continue de reculer le premier coulisseau 76 (figure 4), ce qui fait reculer le second entraînant ainsi les deux fils 30 et 40 vers l'arrière. Le crochet 35 passe ainsi à travers la bague 14, ce qui libère l'implant 10 à l'endroit désiré.

Le tout est de préférence suivi par radiographie.

Si l'on souhaite "récupérer" l'implant 10 après libération, on refait passer le crochet 35 de la première tige 30, puis la seconde tige 40, dans la bague 14, au-delà de son extrémité distale 18. Ensuite, on peut tirer les fils et le cathéter en tenant fixement la gaine (ou pousser sur la gaine 60 en tenant fixement la poignée 70) pour faire rentrer l'implant 10 dans ladite gaine 60 à l'aide du crochet 35 afin qu'il reprenne son état radialement resserré tel que représenté sur la figure 1.

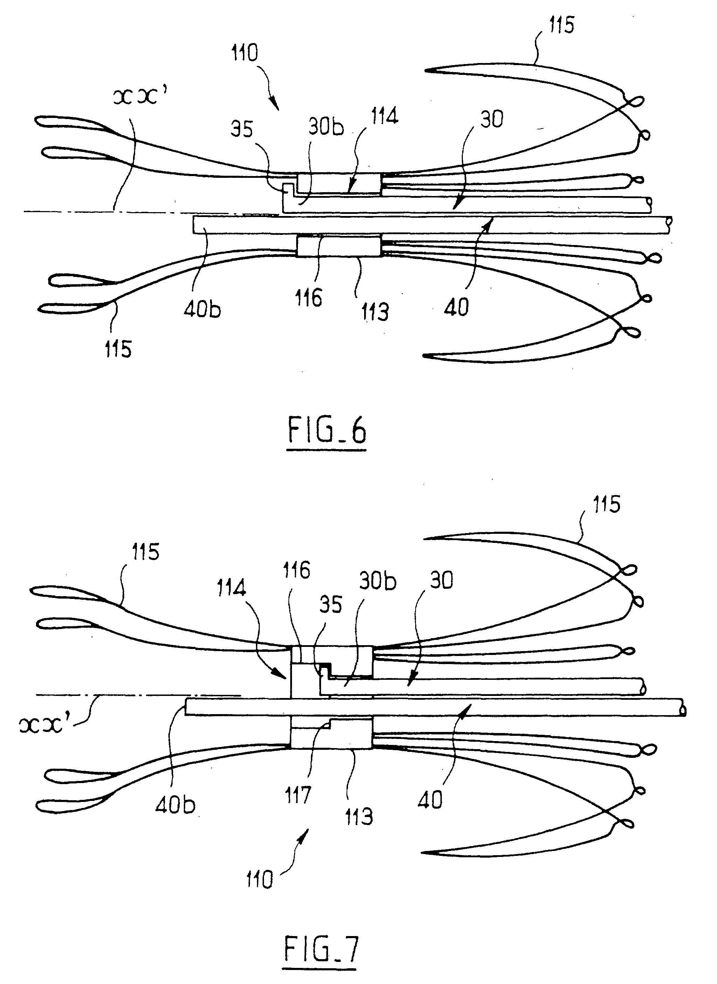

Selon un autre mode de réalisation représenté sur les figures 6 et 7, l'invention peut aussi s'appliquer à l'implantation d'un filtre sanguin 110 comprenant des pattes 115 radialement mobiles, tel en particulier que le filtre temporaire/définitif du brevet FR-A-2 718 949, en l'espèce relié par sa tête 113 au dispositif de retenue/libération 20. Pour cela, la tête 113 est munie d'un orifice 116 débouchant définissant ainsi un tube 114. Cet orifice 116 peut présenter un épaulement 117 contre lequel le crochet 35 peut prendre appui pour retenir le filtre dans son état radialement resserré à l'intérieur de la gaine 60, contre l'extrémité distale 50b du cathéter 50.

La manoeuvre de l'ensemble ainsi constitué est la même que précédemment.

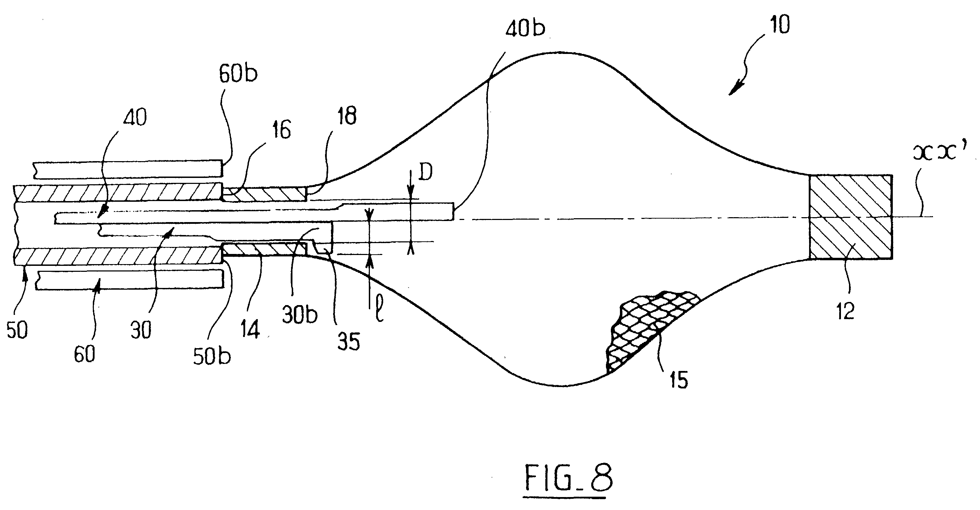

Si l'on se reporte maintenant à la figure 8, on constate que le la section (en l'occurrence le diamètre) d'au moins un (30 ou 40) des deux fils utilisés, et de préférence des deux fils 30 et 40, est strictement inférieure au diamètre interne de la gaine 50 dans laquelle ce(s) fil(s) coulissent, sauf éventuellement, comme cela est représenté, à proximité de leur extrémité distale 30b et 40b où ils ont de préférence un diamètre plus important, avec la présence d'un épaulement à l'endroit du changement de diamètre (une solution à variation progressive du diamètre est également envisageable). Ainsi, la somme des sections des deux fils 30 et 40 est strictement inférieure au diamètre interne du cathéter (50). Cette solution permet de faciliter le mouvement relatif des fils 30 et 40 à l'intérieur de la gaine 50, en évitant un frottement trop important pouvant provoquer un blocage ou un mauvais largage de l'implant. Cette configuration des fils permet également d'assurer un bon accrochage de l'implant 10/110 notamment en raison du fait que la portion de chaque fil située à l'intérieur de la zone tubulaire 14/114 de l'implant, lorsque ce dernier est accroché au dispositif en situation de mise en place, est de section plus importante que le reste du fil, et donc plus rigide.

A noter encore que le crochet 35 peut prendre différentes formes, par exemple en fourche ou en "V".

De même les tiges 30 et 40 peuvent présenter une section en forme de demi cercle, ce qui permet de mieux centrer les fils et d'éviter que le crochet tourne sur lui-même et se positionne mal. On précisera aussi que par éléments allongés, on comprendra qu'il s'agit par exemple de tiges pleines, de cathéters ou de fils (droits ou en hélice).

On peut aussi remplacer la bague 14 par toute zone creuse axialement permettant d'y loger les deux tiges 30 et 40 et l'excroissance 35, dans le but de retenir l'implant 10 à son dispositif de retenue, tout en permettant sa libération le moment voulu.

## Revendications

1. Ensemble pour la mise en place d'un implant (10) dans un conduit interne d'un corps, comprenant :
- l'implant (10), lequel présente un axe (xx') et comprend au moins une zone (14 ; 114) tubulaire axiale ayant un diamètre (D) intérieur, et une structure (15 ; 115) propre à présenter un premier état radialement déployé dans une situation implantée dans le conduit, ou un second état radialement restreint en situation de mise en place,
- et un dispositif (20) de retenue ou de libération de l'implant (10), comprenant au moins un premier (30) et un second (40) éléments allongés amovibles ayant une section déterminée et s'étendant essentiellement parallèlement l'un à l'autre, le premier élément (30) présentant, vers une extrémité distale (30b), une excroissance (35) latérale ayant une longueur radiale (1), tandis que l'autre élément (40) en est dépourvu, la dimension cumulée en section des deux éléments (30, 40) à l'écart de leur extrémité distale (30b, 40b) et la longueur radiale (1) de ladite excroissance latérale (35) étant inférieure au diamètre interne (D) de la zone tubulaire (14 ; 114) de l'implant (10) pour que les éléments (30, 40) passent à travers par leur extrémité distale (30b, 40b), en situation de mise en place de l'implant (10) dans le conduit, tout en étant adaptés pour en être retirés une fois l'implant (10) en place.

2. Ensemble selon la revendication 1, **caractérisé en ce qu'**il comprend un cathéter (50) et **en ce que** les premier (30) et second (40) éléments allongés se présentent comme deux longs fils (30, 40) disposés glissants dans le cathéter (50) avec l'excroissance (35) du premier élément alors située à l'extérieur dudit cathéter (50), le cathéter (50) et les fils (30, 40) ayant une longueur suffisante pour être manoeuvrés depuis l'extérieur du corps, alors que l'implant (10) est dans le conduit.

3. Ensemble selon la revendication 2, **caractérisé en ce qu'**au moins un (30 ou 40) des deux fils présente, sur la majeure partie de sa longueur, une section strictement inférieure à la moitié du diamètre interne du cathéter (50), l'autre fil pouvant présenter, sur toute sa longueur, une section inférieure ou égale à la moitié du diamètre interne dudit cathéter (50), de telle sorte que la somme des sections des deux fils (30 et 40) est strictement inférieure au diamètre interne du cathéter (50).

4. Ensemble selon la revendication 3, **caractérisé en ce que** les deux fils (30 et 40) ont une section circulaire et présentent chacun un diamètre strictement inférieur à la moitié du diamètre interne du cathéter (50) sur toute leur longueur à l'exclusion d'une zone proche de leur extrémité distale (30b/40b) où leur diamètre est supérieur ou égal à la moitié du diamètre interne dudit cathéter (50).

5. Ensemble selon l'une quelconque des revendications 2 à 4, **caractérisé en ce qu'**il comprend en outre une gaine (60) ayant un diamètre interne supérieur au diamètre externe du cathéter (50) et de la zone tubulaire (14 ; 114) de l'implant (10), propre à y loger l'implant (10) dans son état radialement restreint, le cathéter (50) et les fils (30, 40) constituant un moyen de manoeuvre suffisamment rigide pour assurer un déplacement axial de l'implant (10) pour sa mise en place dans le conduit.

6. Ensemble selon l'une quelconque des revendications 2 à 5, **caractérisé en ce que**, en position de tenue de l'implant (10), alors que l'extrémité distale (30b, 40b) des éléments allongés (30, 40) est engagée à travers la zone tubulaire (14) dudit implant (10), le cathéter (50) est placé sensiblement contre une surface proximale (16) de cette zone (14) tandis que l'excroissance latérale (35) du premier élément (30) est placée contre une surface distale (18) de ladite zone (14).

7. Ensemble selon l'une quelconque des revendications 1 à 6, **caractérisé en ce que** le premier élément (30) se présente comme une tige (30) sensiblement rectiligne courbée à son extrémité distale (30b) en faisant un angle compris entre environ 45° et 90° avec cette tige(30) pour former un court crochet (35).

8. Ensemble selon l'une quelconque des revendications 1 à 7, **caractérisé en ce qu'**il comprend en outre une poignée (70) présentant une extrémité avant (72) au-delà de laquelle s'étendent les éléments allongés (30, 40), chaque élément (30, 40) étant fixé à un coulisseau (76, 78) axial, le premier coulisseau (76) auquel est fixé le second élément (40) étant situé devant celui (78) auquel est fixé le premier élément (30).

9. Ensemble selon la revendication 8, **caractérisé en ce que** le premier coulisseau (76) est muni d'un embout (77) de manoeuvre pour être accessible à la main de l'utilisateur, tandis que le second coulisseau (78) est inaccessible de l'extérieur de la poignée (70).

10. Ensemble selon l'une quelconque des revendications précédentes, **caractérisé en ce que** l'implant (10) est un filtre sanguin (110) propre à être implanté soit temporairement, soit définitivement consécutivement au retrait du dispositif (20) de tenue/libération, à l'écart de la zone tubulaire (114) du filtre (110).

11. Ensemble selon l'une quelconque des revendications 1 à 9, **caractérisé en ce que** l'implant (10) est un dispositif (10) d'occlusion ou d'athérectomie vasculaire.

## Patentansprüche

1. Einheit zum Einsetzen eines Implantates (10) in einen inneren Gang eines Körpers, mit:
- einem Implantat (10), das eine Achse (xx') und mindestens einen axialen, röhrenförmigen Bereich (14; 114) mit einem inneren Durchmesser (D) und einen Aufbau (15; 115) aufweist, der geeignet ist, in einer in dem Gang implantierten Lage einen ersten radial entfalteten Zustand oder in eingesetzter Lage einen zweiten radial eingeengten Zustand aufzuweisen,
- und mit einer Vorrichtung (20) zum Zurückhalten oder zur Freigabe des Implantates (10), die mindestens ein erstes (30) und ein zweites (40) längliches, lösbares Element mit einem festgelegten Querschnitt hat, wobei sich die Elemente im wesentlichen parallel zueinander erstrecken, das erste Element (30) zu einem distalen Ende (30b) eine seitliche Ausstülpung (35) aufweist, die eine radiale Länge (I) hat, während das andere Element (40) diese nicht hat, die summierte Abmessung im Querschnitt der zwei Elemente (30, 40) im Abstand von ihrem distalen Ende (30b, 40b) und die radiale Länge (I) der seitlichen Ausstülpung (35) kleiner sind als der innere Durchmesser (D) des röhrenförmigen Bereiches (14; 114) des Implantates (10), damit die Elemente (30, 40) quer durch ihr distales Ende (30b, 40b) in eingesetztem Zustand des Implantates (10) in dem Gang hindurchgehen und zugleich geeignet sind, herausgezogen zu werden, wenn das Implantat (10) einmal eingesetzt ist.

2. Einheit nach Anspruch 1, **dadurch gekennzeichnet, daß** sie einen Katheter (50) aufweist und daß sich das erste (30) und das zweite (40) längliche Element als zwei lange Drähte (30, 40) darstellen, die gleitend in dem Katheter (50) angeordnet sind, wobei die Ausstülpung (35) des ersten Elementes dann außerhalb des Katheters (50) liegt, und der Katheter (50) und die Drähte (30, 40) eine ausreichende Länge haben, um von außerhalb des Körpers betätigt zu werden, wenn sich das Implantat (10) in dem Gang befindet.

3. Einheit nach Anspruch 2, **dadurch gekennzeichnet, daß** mindestens einer der zwei Drähte (30 oder 40) auf dem Hauptteil seiner Länge einen Querschnitt aufweist, der strikt kleiner ist als die Hälfte des inneren Durchmessers des Katheters (50), wobei der andere Draht auf seiner ganzen Länge einen Querschnitt aufweisen kann, der kleiner oder gleich der Hälfte des inneren Durchmessers des Katheters (50) sein kann, derart, daß die Summe der Querschnitte der zwei Drähte (30 und 40) strikt kleiner ist als der innere Durchmesser des Katheters (50).

4. Einheit nach Anspruch 3, **dadurch gekennzeichnet, daß** die zwei Drähte (30 und 40) einen kreisrunden Querschnitt haben und jeder einen Durchmesser aufweist, der strikt kleiner als die Hälfte des inneren Durchmessers des Katheters (50) auf ihrer gesamten Länge ist, mit Ausnahme eines Bereiches nahe ihrem distalen Ende (30b/40b), wo ihr Durchmesser größer oder gleich der Hälfte des inneren Durchmessers des Katheters (50) ist.

5. Einheit nach einem der Ansprüche 2 bis 4, **dadurch gekennzeichnet, daß** sie ferner eine Hülle (60) mit einem inneren Durchmesser aufweist, der größer ist als der äußere Durchmesser des Katheters (50) und des röhrenförmigen Bereiches (14; 114) des Implantates (10), die geeignet ist, hier das Implantat (10) in seinem radial eingeengten Zustand unterzubringen, wobei der Katheter (50) und die Drähte (30, 40) ein ausreichend steifes Betätigungsmittel bilden, um ein axiales Verschieben des Implantates (10) für sein Einsetzen in den Gang sicherzustellen.

6. Einheit nach einem der Ansprüche 2 bis 5, **dadurch gekennzeichnet, daß** in Halteposition des Implantates (10), während das distale Ende (30b, 40b) der länglichen Elemente (30, 40) quer durch den röhrenförmigen Bereich (14) des Implantates (10) in Eingriff ist, der Katheter (50) im wesentlichen gegen eine proximale Oberfläche (16) dieses Bereiches (14) angeordnet ist, während die seitliche Ausstülpung (35) des ersten Elementes (30) gegen eine distale Oberfläche (18) des Bereiches (14) angeordnet ist.

7. Einheit nach einem der Ansprüche 1 bis 6, **dadurch gekennzeichnet, daß** sich das erste Element (30) als ein im wesentlichen gerader Schaft (30) darstellt, der an seinem distalen Ende (30b) gebogen ist, wobei er mit diesem Schaft (30) einen Winkel von zwischen etwa 45° und 90° bildet, um einen kurzen Haken (35) zu formen.

8. Einheit nach einem der Ansprüche 1 bis 7, **dadurch gekennzeichnet, daß** sie ferner einen Griff (70) aufweist, der ein vorderes Ende (72) hat, über das sich die länglichen Elemente (30, 40) erstrecken, wobei jedes Element (30, 40) an einem axialen Schieber (76, 78) befestigt ist, und der erste Schieber (76), an dem das zweite Element (40) befestigt ist, vor dem Schieber (78) liegt, an dem das erste Element (30) befestigt ist.

9. Einheit nach Anspruch 8, **dadurch gekennzeichnet, daß** der erste Schieber (76) mit einem Ansatzstück (77) zur Betätigung versehen ist, um für die Hand des Benutzers erreichbar zu sein, während der zweite Schieber (78) von außerhalb des Griffes (70) nicht zugänglich ist.

10. Einheit nach einem der vorhergehenden Ansprüche, **dadurch gekennzeichnet, daß** das implantat (10) ein Blutfilter (110) ist, der geeignet ist, entweder vorübergehend oder endgültig, bedingt durch das Zurückziehen der Vorrichtung (20) zum Halten/Freisetzen, im Abstand des röhrenförmigen Bereiches (114) des Filters (110) implantiert zu werden.

11. Einheit nach einem der Ansprüche 1 bis 9, **dadurch gekennzeichnet, daß** das Implantat (10) eine vaskuläre Verschluß- oder Atherektomievorrichtung (10) ist.

## Claims

1. Assembly for positioning an implant (10) in an internal passageway of a body, comprising:
- the implant (10), which has an axis (xx') and comprises at least one axial tubular region (14; 114) having an inside diameter (D), and a structure (15; 115) suitable for adopting a first, radially deployed, state in a situation implanted in the passageway, or a second, radially restricted, state in a positioning situation,
- and a device (20) for retaining or releasing the implant (10), comprising at least a first (30) and a second (40) removable elongate element, which elements have a predetermined cross-section and extend substantially parallel to one another, the first element (30) having, towards a distal end (30b), a lateral protuberance (35) having a radial length (1), while the other element (40) has no such protuberance, the cumulative cross-sectional dimension of the two elements (30, 40) away from their distal end (30b, 40b), and the radial length (1) of the lateral protuberance (35), being smaller than the inside diameter (D) of the tubular region (14; 114) of the implant (10) so that the elements (30, 40) pass through at their distal end (30b, 40b) in a situation of positioning the implant (10) in the passageway, while being adapted to be withdrawn therefrom once the implant (10) is in place.

2. Assembly according to claim 1, **characterised in that** it includes a catheter (50) and **in that** the first (30) and second (40) elongate elements are in the form of two long wires (30, 40) arranged slidably in the catheter (50) with the protuberance (35) of the first element then being located outside the catheter (50), the catheter (50) and the wires (30, 40) being sufficiently long to be manoeuvred from outside the body, while the implant (10) is in the passageway.

3. Assembly according to claim 2, **characterised in that** at least one (30 or 40) of the two wires has over most of its length a cross-section which is strictly smaller than half the inside diameter of the catheter (50), it being possible for the other wire to have over its entire length a cross-section smaller than or equal to half the inside diameter of the catheter (50), so that the sum of the cross-sections of the two wires (30 and 40) is strictly smaller than the inside diameter of the catheter (50).

4. Assembly according to claim 3, **characterised in that** the two wires (30 and 40) have a circular cross-section and each has a diameter strictly smaller than half the inside diameter of the catheter (50) over their entire length with the exception of a region close to their distal end (30b/40b) where their diameter is greater than or equal to half the inside diameter of the catheter (50).

5. Assembly according to any one of claims 2 to 4, **characterised in that** it also comprises a sleeve (60) which has an inside diameter which is greater than the outside diameter of the catheter (50) and of the tubular region (14; 114) of the implant (10) and which is suitable for accommodating the implant (10) in its radially restricted state, the catheter (50) and the wires (30, 40) constituting a means of manoeuvre sufficiently rigid to provide for axial movement of the implant (10) in order to position it in the passageway.

6. Assembly according to any one of claims 2 to 5, **characterised in that**, when the implant (10) is in its held position, while the distal end (30b, 40b) of the elongate elements (30, 40) is engaged through the tubular region (14) of the implant (10), the catheter (50) is placed substantially against a proximal surface (16) of that region (14) while the lateral protuberance (35) of the first element (30) is placed against a distal surface (18) of said region (14).

7. Assembly according to any one of claims 1 to 6, **characterised in that** the first element (30) is in the form of a substantially rectilinear rod (30) which is curved at its distal end (30b), forming an angle of approximately from 45° to 90° with the rod (30), to form a short hook (35).

8. Assembly according to any one of claims 1 to 7, **characterised in that** it also comprises a grip (70) having a front end (72) beyond which the elongate elements (30, 40) extend, each element (30, 40) being secured to an axial slide (76, 78), and the first slide (76), to which the second element (40) is secured, being arranged in front of that (78) to which the first element (30) is secured.

9. Assembly according to claim 8, **characterised in that** the first slide (76) is provided with a manoeuvring arm (77) in order to be accessible to the user's hand, while the second slide (78) is not accessible from outside the grip (70).

10. Assembly according to any one of the preceding claims, **characterised in that** the implant (10) is a blood filter (110) suitable for being implanted either temporarily or permanently, after the withdrawal of the holding/release device (20) from the tubular region (114) of the filter (110).

11. Assembly according to any one of claims 1 to 9, **characterised in that** the implant (10) is a vascular atherectomy or occlusion device (10).
